# EUROPÄISCHE PATENTSCHRIFT

(11) **EP 1 654 710 B1**
(45) Veröffentlichungstag und Bekanntmachung des Hinweises auf die Patenterteilung: **27.05.2020**
(21) Anmeldenummer: 04737035.8
(22) Anmeldetag: 18.06.2004
(51) Int. Cl.: G06T 15/10

(54) **Darstellung von 3D-Bilddaten**
Representation of 3D Image Data
Présentation de données d'image 3D

(30) Priorität: 15.08.2003 DE 10338145
(43) Veröffentlichungstag der Anmeldung: 10.05.2006
(73) Patentinhaber: siCAT GmbH & Co. KG, 53177 Bonn (DE)
(72) Erfinder: LIÉVIN, Marc, 53129 Bonn (DE); HEY, Joachim, 53332 Bornheim (DE); KEEVE, Erwin, 53115 Bonn (DE)
(74) Vertreter: Braun-Dullaeus, Karl-Ulrich
(86) Internationale Anmeldenummer: PCT/EP2004/006568
(87) Internationale Veröffentlichungsnummer: WO 2005/027050

(56) Entgegenhaltungen:
- DELEGACZ A; LO S -C B; XIE H; FREEDMAN M T; CHOI J J: "Three-dimensional visualization system as an aid for lung cancer" MEDICAL IMAGING 2000, IMAGE DISPLAY AND VISUALIZATION, Bd. 3976, 13. Februar 2000 (2000-02-13), Seiten 401-409, XP002320383 PROCEEDINGS OF THE SPIE - THE INTERNATIONAL SOCIETY FOR OPTICAL ENGINEERING
- CHENG-SHENG HUNG, CHIEN-FENG HUANG, MING OUHYOUNG: "Fast Volume Rendering for Medical Image Data" PROCEEDINGS OF REAL-TIME AND MEDIA SYSTEMS, RAMS, [Online] 31. Dezember 1998 (1998-12-31), Seiten 49-55, XP002320761 TAIPEI, TAIWAN Gefunden im Internet: URL:http://www.csie.ntu.edu.tw/~r7526011/i nfo/mri.html> [gefunden am 2005-03-04]
- MOZZO P; PROCACCI C; TACCONI A; MARTINI P T; ANDREIS I A: "A new volumetric CT machine for dental imaging based on the cone-beam techique: preliminary results" EUROPEAN RADIOLOGY, Bd. 8, Nr. 9, 31. Dezember 1998 (1998-12-31), Seiten 1558-1564, XP002320382 SPRINGER VERLAG, DEUTSCHLAND
- ENGEL K ET AL INSTITUTE OF ELECTRICAL AND ELECTRONICS ENGINEERS: "Combining local and remote visualization techniques for interactive volume rendering in medical applications" PROCEEDINGS VISUALIZATION 2000. VIS 2000. SALT LAKE CITY, UT, OCT. 8 - 13, 2000, ANNUAL IEEE CONFERENCE ON VISUALIZATION, LOS ALAMITOS, CA : IEEE COMP. SOC, US, 8. Oktober 2000 (2000-10-08), Seiten 449-452,587, XP010524637 ISBN: 0-7803-6478-3

## Beschreibung

Die vorliegende Erfindung betrifft die Präsentation von Bilddaten, die ein dreidimensionales Objekt in einem Raum repräsentieren, wobei aus den Bilddaten durch Überlagerung mehrerer Bildebenen Projektionsdaten erzeugt werden, die eine zweidimensionale Projektion des Objektes repräsentieren, und wobei die Projektion auf einem Bildschirm einem Nutzer zur Ansicht dargestellt wird.

Moderne Bilderzeugungssysteme, wie sie insbesondere in der Medizin eingesetzt werden, produzieren Bilddaten (3D Volumendaten), die das abzubildende Objekte in seiner Dreidimensionalität repräsentieren und die für den Nutzer respektive den Betrachter aufbereitet und dargestellt werden müssen. Dabei besteht mittlerweile die Möglichkeit, auf präoperativ oder intraoperativ gewonnene 3D-Bilddaten des zu behandelnden Objekts, beispielsweise eines Kiefers zurückzugreifen, um eine Diagnose durchzuführen und auch um vor dem Eingriff Planungen vornehmen zu können. In der Medizin werden Computertomographen (CT) und Kernspintomographen (MR) als 3D bilderzeugende Geräte eingesetzt. Insbesondere im Dentalbereich wird auch die digitale Volumen Tomographie (DVT) eingesetzt, die sich eines kegelförmigen Strahlenbündels ("Cone Beam") bedient. Dabei werden mit einer den Patienten umkreisenden Anordnung aus Röntgenröhre und Detektor, einem C-Bogen, eine Vielzahl (ca. 100)_von Aufnahmen aus verschiedenen Winkeln gemacht und aus diesen Röntgenaufnahmen ein 3D Bilddatensatz mit bekannten Rechenverfahren ermittelt. Dabei ist die Auflösung des entstandenen 3D Bilddatensatzes kleiner als 0.5 mm.

Die Darstellung der 3D Bilddaten erfolgt meist auf herkömmlichen Bildschirmen und damit in nur zwei Dimensionen (2D). Aus der Chirurgie ist es jedoch bekannt, gleichzeitig drei zueinander orthogonale Schnitte durch das Objekt in zwei Dimensionen darzustellen. Mit einem Zeigeinstrument hat der Bediener dabei die Möglichkeit, die jeweilige Lage der Schnitte respektive die Tiefe der Schnitte vorzugeben. Eine andere Darstellungsmöglichkeit ist die Projektion der 3D Bilddaten auf eine definierte Ebene und eine anschließende 2D Darstellung dieser Ebene.

Nachteilig an den bekannten Verfahren ist, dass bei jeder Projektion von 3D Bilddaten in eine Ebene Detailinformationen insofern verloren gehen, als in Richtung der Projektion über die einzelnen Volumeninformationen gemittelt wird. Im Falle der Darstellung eines 3D Volumens vermittels der orthogonalen Schnitte ist zudem die Handhabbarkeit verhältnismäßig aufwendig. Eine zur Orientierung notwendige Übersichtsdarstellung kann gleichzeitig nur in einem separatem Fenster über eine Volumenprojektion oder eine Oberflächendarstellung des Objekts erfolgen.

Aus Delegaz A et al.:"Three-dimensional visualisation system as an aid for lung cancer" in Medical Imaging 2000, Image Display and Visualisation, Bd. 3976 ist die Kombination verschiedener Rendering-Techniken zur besseren Visualisierung von 3-D Abbildungen einer Lunge bekannt.

Sheng-Sheng Hung et al.: Fast Volume Rendering for Medical Image Data" in Proceedings of real-time and media systems, rams zeigt das Schneiden innerhalb einer Volumengraphik zur Darstellung eines ansonsten verdeckt bleibenden Teilbereichs.

Aufgabe der vorliegenden Erfindung ist es, ein Verfahren zur Präsentation von Bilddaten zu schaffen, das sich einfach und mit preiswerten Mitteln realisieren lässt, und das sich bei hoher Zuverlässigkeit durch eine große Praktikabilität auszeichnet. Gleichsam ist es Aufgabe der Erfindung, ein System zur Umsetzung des Verfahrens zu schaffen.

Diese Aufgaben werden durch das Verfahren mit den kennzeichnenden Merkmalen des Anspruch 1 und das System nach Anspruch 15 gelöst.

Besondere Ausführungsformen sind in den jeweiligen Unteransprüchen genannt.

Der Kerngedanke der Erfindung liegt darin, einerseits die Projektion, die ja von relativ geringer räumlicher Auflösung ist, als Gesamtaufnahme darzustellen. An dieser kann sich der Betrachter orientieren. Andererseits wird dem Nutzer die Möglichkeit zu geben, aus der Projektion einen Ausschnitt auszuwählen und in diesem eine Detailaufnahme höherer respektive anderer Qualität darzustellen. Auf diese Art weiß der Nutzer anhand der Gesamtaufnahme, wo er sich innerhalb des Objektes befindet und bekommt zudem über die Detailaufnahme weitere Information bezüglich des Ortes seines Interesses. Erfindungsgemäß wird dazu aus der Projektion ein Teilbereich ausgewählt, innerhalb des Teilbereiches ein Detailbild erzeugt und das Detailbild dann auf dem Bildschirm dargestellt.

In einer besonders zu bevorzugenden Ausführungsform der Erfindung wird das Detailbild in unmittelbarem oder in mittelbarem Rückgriff auf die Bilddaten erzeugt, aus denen auch die Projektion erzeugt ist, wobei diese Bilddaten in einem ersten Datensatz zusammengefasst sind. Diese Bilddaten wurden zunächst erzeugt, wobei eine Vielzahl von Einzelaufnahmen mit einem der bekannten Verfahren, insbesondere mit einem mobilen C-Bogen oder einem dentalen Panoramagerät, aufgenommen wurden und wobei die Einzelaufnahmen mit einem Computerprogramm verarbeitet wurden.

Das erfindungsgemäße Detailbild zeichnet sich dadurch aus, dass es einen anderen Informationsgehalt aufweist als die Projektion, wobei der höhere Informationsgehalt sich beispielsweise beziehen kann auf die Auflösung und/oder den Blickwinkel und/oder die Tiefeninformation. Auf dem Bildschirm wird dann im Rahmen des vom Nutzer ausgewählten Teilbereiches dieses Detailbild dargestellt. Der Nutzer sieht somit ein Bild im Bild.

Die Erfindung macht es sich dabei zunutze, dass ein 3D Datensatz vorhanden ist, aus dem die Projektionsdarstellung berechnet wird. So stand die eigentliche 3D Information zwar schon immer zur Verfügung, wurde aber bislang nicht dargestellt. Die Berechnung der Projektionsdarstellung ist dabei im Prinzip lediglich eine gewichtete Überlagerung und Mittelung über viele Ebenen. Die für eine Ebene spezifische Information wird dabei naturgemäß verwaschen. Entsprechend der Erfindung wird die dem Nutzer gewohnte Projektionsdarstellung nunmehr so modifiziert, dass ihm innerhalb des ausgewählten Teilbereiches Zugang zu den detaillierteren 3D Informationen des Datensatzes verschafft wird, ohne dass es der bislang üblichen zusätzlichen Darstellung in einem separaten Fenster auf dem Bildschirm bedarf. Es ist dabei von besonderem Vorteil, dass die Bedienung intuitiv und interaktiv erfolgen kann und dass die Übersichtlichkeit der Projektionsdarstellung erhalten bleibt. Das Anwendungsgebiet der Erfindung ist nicht auf die Medizin beschränkt. So ist auch ein Einsatz im nichtmedizinischen Bereich möglich. Als Beispiel seien dabei Röntgenscanner auf Flughäfen genannt.

Vorteilhafterweise wird die Erfindung so umgesetzt, dass in der gewohnten Projektionsdarstellung ein Teilbereich markiert wird, den der Benutzer mit einem Zeigeinstrument, beispielsweise einer Maus, einem Track-Ball oder einem Joystick, an die Stelle schiebt, für die er detailliertere Informationen in 3D sehen möchte. Der Nutzer definiert dazu die Größe und den Ort des Teilbereiches auf dem Bildschirm, wobei er in einer besonderen Ausführungsform auch noch die Kontur der Umrandung selber bestimmen kann. In diesem Teilbereich wird dem Benutzer beispielsweise statt der gesamten Projektion eine dünnere Schicht oder sogar nur eine einzige Schnittebene dargestellt. Der Nutzer könnte auch nach der Platzierung durch die Bewegung des Zeigeinstrumentes für den Bereich parallel zur Darstellungsebene "surfen", also je nach Lage des Zeigeinstruments mehr oder weniger tief in das 3D Volumen eintauchen. Es ist auch möglich, in jeder beliebigen anderen Orientierung, beispielsweise auch transversal, das hinter dem selektierten Bereich "versteckt" liegende Volumen durchzublättern. Der Nutzer hat somit, wie dargestellt, in einer vorteilhaften Ausführungsform interaktiven Zugriff auf eine Vielzahl unterschiedlicher die Bild-Informationen innerhalb des Teilbereiches, wobei er durch Bedienung des Zeigeinstrumentes zwischen verschiedenen Darbietungen, beispielsweise Schichten, die insbesondere parallel zur Projektionsebenen liegen, "blättern" kann.

In einer vorteilhaften Ausführungsform kann der Nutzer unter mehreren möglichen Detailbildern eines auswählen, wobei sich die Detailbilder im Informationsgehalt, insbesondere in der durch das Detailbild repräsentierten Tiefe, in dem Blickwinkel und/oder in der Darstellungsart, unterscheiden. Die Detailbilder können auch von "Sub-Projektionen" gebildet sein. Als derartige Sub-Projektion wird eine Projektion verstanden, die aus einer Zusammenfassung einer Anzahl von Bildschnitten erzeugt ist, wobei diese Anzahl jedoch kleiner ist als die Anzahl der Bildschnitte, aus denen die zur Übersicht dienende Projektion erzeugt ist. Dabei kann die Anzahl im Bereich zwischen eins und der Anzahl aller vorhandenen Schichten minus eins sein. Auf jeden Fall zeichnet sich eine solche Sub-Projektion durch eine höhere Tiefenschärfe von der Projektionen aus, da bei der Erzeugung der Sub-Projektionen weniger Bildebenen überlagert werden. Wenn genau eine Bildebene eine Sub-Projektion repräsentiert, ist die Tiefenschärfe am größten. Letztendlich wird bei der Erzeugung der Sub-Projektion gezielt eine bestimmte Information aus den vorhandenen Bilddaten herauspräpariert.

Zwar ist es rechnerisch möglich, für die Sub-Projektion jede beliebige Blickrichtung zu wählen, aus Gründen der Übersichtlichkeit ist es jedoch vorteilhaft, die Ebene der Sub-Projektionen parallel oder transversal zur Ebene der Projektion zu legen. Durch ein kontinuierliches "Durchblättern" kann der Nutzer ein Objekt im Raum erforschen und dessen Lage begreifen. Dabei kann er auch Strukturen entdecken, die hintereinander liegen und deren räumliche Anordnung ihm in einer Projektionsdarstellung verborgen bliebe. Diese Möglichkeit ist gerade im Fall von geplanten Zahnbehandlungen vorteilhaft, da damit alle gefährdeten Strukturen entdeckt und in ihrem absoluten Abstand vermessen werden können.

In einer weiteren vorteilhaften Ausführungsform wird das Detailbild in Rückgriff auf Bilddaten erzeugt, die in einem zweiten Datensatz zusammengefasst sind, wobei diese Bilddaten einer anderen Aufnahme des Objektes entstammen. Diese Bilddaten des zweiten Datensatzes können insbesondere mit einem anderen Gerät oder mit demselben Gerät zu einem anderen Zeitpunkt (z.B. prä- oder postoperativ) oder auch mit demselben Gerät aber anderen Geräteparametern aufgenommen worden sein. Bei der Überlagerung und Anpassung verschiedener Sätze von Bilddaten werden vorteilhaft Bildinformationen mit bekannten Verfahren in Übereinstimmung gebracht. Solche Verfahren sind beispielsweise bekannt aus "A Survey of Medical Image Registration", von J.B. Antoine Maintz und Max Viergever, Medical Image Analysis, Vol.2, pp 1-36, 1998. Mit der Möglichkeit, innerhalb des Detailbildes auch aus anderen Datenbeständen zu schöpfen, lassen sich für den Nutzer zeitliche Veränderungen verfolgen oder interessante Details mit einer entsprechend des anderen Aufnahmemodus höheren Qualität darstellen. So kann die aus den Detailbildern entnommene Information noch weiter erhöht werden. Dabei können innerhalb des Detailbildes auch Überlagerungen verschiedener Datensätze dargestellt werden.

Weiterhin ist es vorteilhaft, ein separates Fenster auf dem Bildschirm vorzusehen, das außerhalb der Projektionsdarstellung geöffnet werden kann und in dem verschiedene Schnitte durch das Objekt im Rahmen des zuvor ausgewählten Teilbereiches dargestellt werden. So kann der Nutzer sich einen Überblick über das vorhandene Angebot darstellbarer Schnitte verschaffen, bevor er unter diesen Schnitten auswählt. Statt der Schnitte kann in dem separaten Fenster auch eine 3D Volumendarstellung des Details oder eine Oberflächendarstellung erfolgen. Bezüglich derartiger Möglichkeiten sind der Erfindung nur Grenzen gesetzt durch den Bestand der Bilddaten und durch die zur Verfügung stehende Rechnerkapazität.

Ein besonders vorteilhaftes Anwendungsgebiet der Erfindung liegt in der Medizin, wobei die Bilddaten einen Teil eines menschlichen oder tierischen Körpers repräsentieren und mit einem Diagnosesystem aufgenommen sind. Gerade in der Zahnheilkunde ist der Einsatz des Verfahrens besonders vorteilhaft. Wie schon angesprochen, ist es zudem vorteilhaft, wenn es sich um Bilddaten handelt, die mit einem Computertomographen, einem Kernspintomographen, einem digitalen Volumen Tomographen oder vor allem mit einem insbesondere mobilen C-Bogen aufgenommen worden sind.

Nachfolgend wird die Erfindung anhand der Figuren 1 bis 4 näher beschrieben. Es zeigen:
- **Figur 1**: ein Schema der Aufarbeitung der Bilddaten,
- **Figur 2**: eine Projektion eines Kiefers,
- **Figur 3**: die Projektion des eines Kiefers mit Detaildarstellung im Teilbereich und
- **Figur 4**: a-c, Projektionen des Kiefers mit jeweils einer anderen Detaildarstellung im Teilbereich.

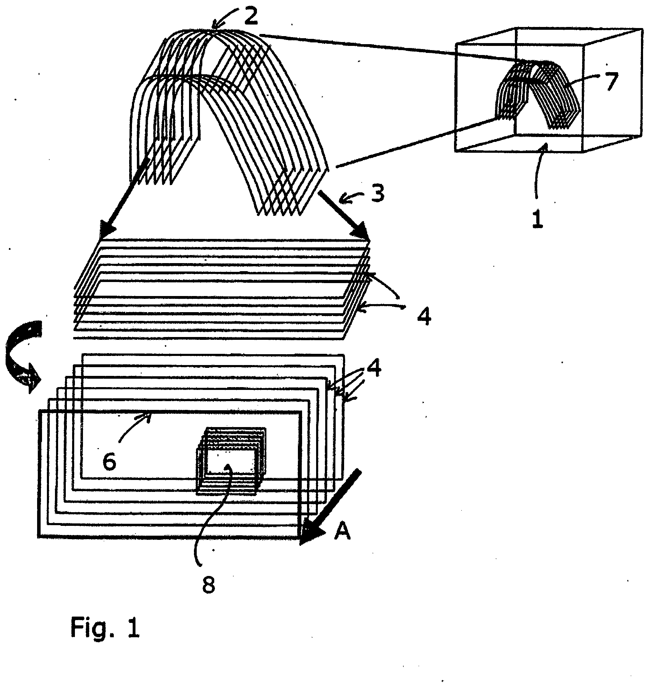

In den Figuren ist das Verfahren zur Präsentation von Bilddaten dargestellt. Dabei wurde zunächst mittels eines bekannten Diagnoseverfahrens (CT, MR, DVT o.ä.) ein Satz von Bilddaten 1 aufgenommen, der das Objekt, hier einen menschlichen Kiefer 7, im Raum repräsentiert. Die Bilddaten 1 sind symbolisch innerhalb eines als Quader dargestellten Speicherbereiches in der Figur 1 dargestellt. Als Ergebnis der Aufnahme entsteht somit ein Bilddatenkubus mit beispielsweise 256³ Bildpunkten.

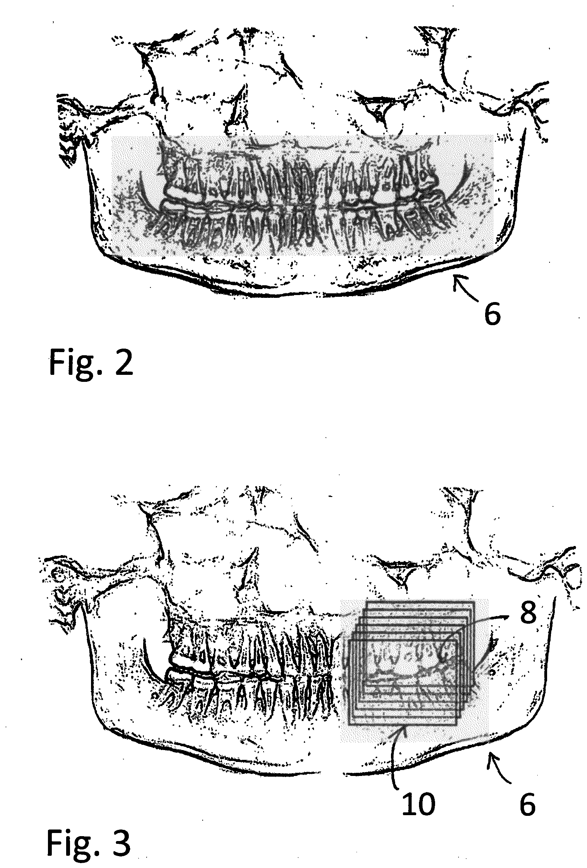

Für die Bilderzeugung werden Teile 2 der Bilddaten 1, hier im wesentlichen die den Kiefer im Raum beschreibenden Bildpunkte, die auf gekrümmten Ebenen entlang des Kieferkamms liegen, herausgegriffen, bevor aus diesen Teilen 2 letztendlich eine Projektion 6 (Figur 2) erzeugt wird. Zur Erstellung der Projektion 6 werden die extrahierten Teile 2 in einem weiteren Verfahrensschritt 3 in ihrer Dreidimensionalität derart aufgespannt, dass hintereinanderliegende zweidimensionale Bildebenen 4 entstehen. Diese Ebenen 4 werden in Richtung hin zum Betrachter gedreht 5 und dann zu der Projektion 6 aufsummiert (Figur 1, Pfeil A), wobei der mit starker Strichstärke gezeichnete Rahmen die Projektion darstellt. Durch die Addition geht jedoch die den einzelnen Bildebenen 4 anhaftende Information verloren, da über die gesamte Tiefe gemittelt wird. Aus den Bilddaten werden letztendlich durch rechnerische Überlagerung mehrerer Bildebenen 4 Projektionsdaten erzeugt, die eine zweidimensionale Projektion 6 des Objektes 7 repräsentieren, wobei die Projektion 6 auf einem Bildschirm einem Nutzer zur Ansicht dargestellt wird. Selbstverständlich werden die gesamten Verarbeitungsschritte betreffend die Bildinformation von einem Computer vorgenommen.

Erfindungswesentlich ist, dass nun innerhalb der Projektion 6 ein Teilbereich 8 ausgewählt wird. Dies geschieht beispielsweise mittels einer "Maus" und eines von der Maus gesteuerten und von einem Rechteck umgebenen Cursors auf dem Bildschirm. So kann mittels einer Maustaste der Bereich 8 in Größe und/oder Ort auf dem Bildschirm definiert werden. Innerhalb des gewählten Teilbereiches 8 wird unmittelbar oder mittelbar auf die Bilddaten 1 zurückgegriffen und ein Detailbild 9 (Figur 3) erzeugt, das sich gegenüber der Projektion 6 durch eine andere Bildqualität respektive durch einen anderen Informationsgehalt auszeichnet. In diesem Fall werden die einzelnen in der Projektion 6 summierten Bildebenen 4 wieder aufgelöst und können separat dargestellt werden. Sie bilden damit Sub-Projektionen 10 höherer Tiefenschärfe.

In Figur 2 ist der Stapel der hintereinanderliegenden Sub-Projektionen 10 innerhalb der Projektion 6 dargestellt. Mit einer anderen Mausfunktion kann der Stapel Schicht für Schicht "durchgeblättert" werden. Durch einfache Bewegung der Maus bekommt der behandelnde Arzt ein Gefühl für die Dreidimensionalität der Zähne und kann seine Behandlung den tatsächlichen Gegebenheiten anpassen. Der Arzt sieht auf einmal Dinge, die ihm bis dahin verborgen waren. Dadurch kann das Risiko der Behandlung stark reduziert werden.

Anders ausgedrückt liegt auf dem Computer ein 3D Volumendatensatz 1, während eine Software die Projektion 6 entlang einer gewünschten Richtung berechnet. Die Projektion 6 wird dann auf dem Monitor darstellt. Die Software hat dabei Zugriff auf den gesamten 3D Datensatz und kann jede beliebige Schicht, beispielsweise jede zur Projektionsebene parallel verlaufende Schicht, in dem Teilbereich darstellen. Der Benutzer kann dann durch die Bewegung des Zeigeinstruments interaktiv in den Schichten, also senkrecht zur dargestellten Ebene, blättern.

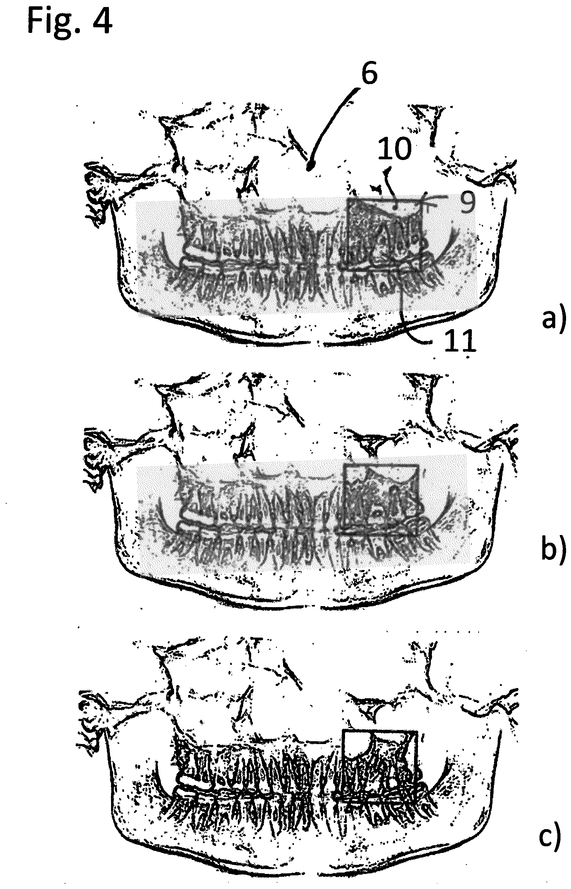

In Figur 4 sind dargestellt jeweils eine Projektion 6, die den Großteil des Bildschirms einnimmt. Innerhalb jeder Projektion ist ein Detailbild 9 dargestellt, das jeweils eine andere Schicht innerhalb des Kiefers zeigt. Deutlich zu erkennen ist die Schichtung des Fünfer Backenzahnes links oben 11, von dem in Figur 4c die beiden außenliegenden Wurzeln zu sehen sind, während in Figur 4a die innenliegende Wurzel zu erkennen ist. In der Projektion sind diese Details nicht auflösbar.

Die hier dargestellten Bilder eines Kiefers wurden mit einem C-Bogen der bekannten Art aufgenommen. Dabei bediente sich das Verfahren zu Erzeugung aus den Einzelaufnahmen ermittelten Schichtbilder der ebenfalls bekannten Algorithmen.

## Patentansprüche

1. Verfahren zur Präsentation von Bilddaten (1), die ein dreidimensionales Objekt (7) in einem Raum repräsentieren, wobei aus den Bilddaten (1) durch rechnerische Überlagerung mehrerer Bildebenen Projektionsdaten erzeugt werden, die eine zweidimensionale Projektion (6) des Objektes (7) repräsentieren, und wobei die Projektion (6) auf einem Bildschirm einem Nutzer zur Ansicht dargestellt wird,
**dadurch gekennzeichnet,**
**dass** innerhalb der Projektion (6) ein Teilbereich (8) ausgewählt wird,
**dass** innerhalb des Teilbereiches (8) ein Detailbild (9) erzeugt wird, das eine andere Tiefe und/oder Blickwinkel und/oder Darstellungsart und/oder Tiefeninformation aufweist als die Projektion (6) und dass das Detailbild (9) auf dem Bildschirm innerhalb des Teilbereiches (8) dargestellt wird.

2. Verfahren nach Anspruch 1,
**dadurch gekennzeichnet,**
**dass** das Detailbild in unmittelbarem oder in mittelbarem Rückgriff auf die Bilddaten (1) erzeugt wird, aus denen die Projektion erzeugt ist, wobei diese Bilddaten (1) in einem ersten Datensatz zusammengefasst sind.

3. Verfahren nach einem der vorherigen Ansprüche,
**dadurch gekennzeichnet,**
**dass** ein Detailbild (9) eine Sub-Projektion (10) ist, die sich durch eine höhere Tiefenschärfe von der Projektionen (6) unterscheidet.

4. Verfahren nach Anspruch 3,
**dadurch gekennzeichnet,**
**dass** die Ebenen (4) der Sub-Projektionen (10) parallel zur Ebene der Projektion (6) ist.

5. Verfahren nach einem der vorherigen Ansprüche,
**dadurch gekennzeichnet,**
**dass** auf dem Bildschirm ein separates Fenster eröffnet wird, in dem verschiedene Schnitte durch das Objekt (7) im Rahmen des ausgewählten Teilbereiches (8) dargestellt werden.

6. Verfahren nach einem der vorherigen Ansprüche,
**dadurch gekennzeichnet,**
**dass** in dem separaten Fenster eine Volumendarstellung oder eine Oberflächendarstellung erfolgt.

7. Verfahren nach einem der vorherigen Ansprüche,
**dadurch gekennzeichnet,**
**dass** bei der Erzeugung der Sub-Projektionen (10) mit höherer Tiefenschärfe weniger Bildebenen (4) überlagert werden als bei der Erzeugung der Projektionen (6).

8. Verfahren nach einem der vorherigen Ansprüche,
**dadurch gekennzeichnet,**
**dass** genau eine Bildebene (4) eine Sub-Projektion (10) repräsentiert.

9. Verfahren nach einem der vorherigen Ansprüche,
**dadurch gekennzeichnet,**
**dass** der Nutzer innerhalb des Teilbereiches (8) interaktiven Zugriff auf die Bild-Informationen hat, indem er durch Bewegen eines Zeigeinstrumentes zwischen verschiedenen Schichten parallel zur Projektionsebenen blättern kann.

10. Verfahren nach einem der vorherigen Ansprüche,
**dadurch gekennzeichnet,**
**dass** die Bilddaten einen Teil eines menschlichen oder tierischen Körpers repräsentieren und mit einem Diagnosesystem aufgenommen sind.

11. Verfahren nach Anspruch 10,
**dadurch gekennzeichnet,**
**dass** die Bilddaten mit einem Computertomographen (CT), einem Kernspintomographen (MR) oder der digitalen Volumen Tomographie (DVT) aufgenommen sind.

12. Verfahren nach Anspruch 10,
**dadurch gekennzeichnet,**
**dass** die Bilddaten mit einem C-Bogen aufgenommen sind, der um das Objekt rotiert wurde.

13. Verfahren nach einem der vorherigen Ansprüche,
**dadurch gekennzeichnet,**
**dass** das Detailbild in unmittelbarem oder in mittelbarem Rückgriff auf Bilddaten erzeugt wird, die in einem zweiten Datensatz zusammengefasst sind, wobei diese Bilddaten einer anderen Aufnahme des Objektes entstammen.

14. Verfahren nach Anspruch 13
**dadurch gekennzeichnet,**
**dass** die Bilddaten des zweiten Datensatzes mit einem anderen Gerät, zu einem anderen Zeitpunkt oder mit anderen Geräteparametern aufgenommen wurden.

15. System zur Umsetzung des Verfahrens nach einem der vorherigen Ansprüche,
**gekennzeichnet durch**
einen Computer mit Zugriff auf die Bilddaten, wobei auf dem Computer ein Programm realisiert ist, das aus den Bilddaten Schnittbilder extrahiert und zu der Projektion (6) überlagert,
einen Bildschirm, auf dem die Projektion (6) darstellbar ist,
Mitteln, die dem Nutzer die Möglichkeit geben, innerhalb der Projektion (6) einen Teilbereich (8) zu definieren, wobei das Programm eine Funktion umfasst, die in mittelbarem oder unmittelbaren Rückgriff auf die Bilddaten (1) ein Detailbild (9) erzeugt, wobei das Detailbild (9) eine andere Tiefe und/oder Blickwinkel und/oder Darstellungsart und/oder Tiefeninformation Informationsgehalt aufweist als die Projektion (6) und auf dem Bildschirm innerhalb des Teilbereiches (8) dargestellt wird.

16. System nach Anspruch 15,
**gekennzeichnet durch**
Mittel, mit denen der Nutzer eines unter verschiedenen Detailbildern (9) auswählt.

17. Verwendung des Verfahrens nach einem der vorherigen Verfahrensansprüche zur Präsentation von Bilddaten im Bereich der Zahnheilkunde.

## Claims

1. A method for presenting image data (1), which data represent a three-dimensional object (7) in a space, wherein projection data, which represent a two-dimensional projection (6) of the object (7), are generated from the image data (1) by computationally superimposing multiple image planes, and wherein the projection (6) is displayed for viewing to a user on a screen,
**characterized in that**
a subarea (8) is selected within the projection (6), that a detailed image (9), which has a depth and/or perspective and/or form of display and/or depth information other than that/those of the projection (6), is generated within the subarea (8) and that the detailed image (9) is displayed within the subarea (8) on the screen.

2. The method according to Claim 1,
**characterized in that**
the detailed image is generated by directly or indirectly accessing the image data (1) from which the projection is generated, wherein these image data (1) are combined in a first data set.

3. The method according to any one of the preceding claims,
**characterized in that**
a detailed image (9) is a sub-projection (10), which differs from the projections (6) by a higher depth of field.

4. The method according to Claim 3,
**characterized in that**
the planes (4) of the sub-projections (10) are parallel to the plane of the projection (6).

5. The method according to any one of the preceding claims,
**characterized in that**
a separate window is opened on the screen, in which different sections through the object (7) are represented within the selected subarea (8).

6. The method according to any one of the preceding claims,
**characterized in that**
a volumetric representation or a surface representation is displayed in the separate window.

7. The method according to any one of the preceding claims,
**characterized in that**
fewer image planes (4) are superimposed in the generation of the sub-projections (10) with higher depth of field than in the generation of the projections (6) .

8. The method according to any one of the preceding claims,
**characterized in that**
exactly one image plane (4) represents a sub-projection (10) .

9. The method according to any one of the preceding claims,
**characterized in that**
the user has interactive access to the image information within the subarea (8) **in that** by moving a pointer instrument, he can scroll between different layers parallel to the projection planes.

10. The method according to any one of the preceding claims,
**characterized in that**
the image data represent a part of a human or animal body and are recorded with a diagnostic system.

11. The method according to Claim 10,
**characterized in that**
the image data are recorded with a computer tomography (CT) scanner, with a magnetic resonance (MRI) scanner or with digital volume tomography (DVT).

12. The method according to Claim 10,
**characterized in that**
the image data are recorded with a C-arm, which was rotated around the object.

13. The method according to any one of the preceding claims,
**characterized in that**
the detailed image is generated by directly or indirectly accessing image data that are combined in a second data set, wherein these image data originate from another recording of the object.

14. The method according to Claim 13,
**characterized in that**
the image data of the second data set were recorded with a different device, at a different time or with different device parameters.

15. A system for implementing the method according to any one of the preceding claims,
**characterized by**
a computer having access to the image data, wherein a program, which extracts sectional images from the image data and superimposes them to form the projection (6), is run on the computer,
a screen on which the projection (6) can be displayed,
means which enable the user to define a subarea (8) within the projection (6), wherein the program comprises a function which generates a detailed image (9) by indirectly or directly accessing the image data (1), wherein the detailed image (9) has a depth and/or perspective and/or form of display and/or depth information/information content other than that/those of the projection (6) and is displayed within the subarea (8) on the screen.

16. The system according to claim 15,
**characterized by**
means with which the user selects one from among different detailed images (9).

17. A use of the method according to any one of the preceding method claims for the presentation of image data in the dentistry field.

## Revendications

1. Procédé de présentation de données-images (1), qui représentent un objet tridimensionnel (7) dans un espace, sachant qu'à partir des données-images (1) des données de projection sont produites par superposition numérique de plusieurs plans d'image, qui représentent une projection bidimensionnelle (6) de l'objet (7) et sachant que la projection (6) est représentée sur un écran à un utilisateur pour avis,
**caractérisé en ce qu'**
à l'intérieur de la projection (6), une partie de zone (8) est sélectionnée,
**en ce qu'**à l'intérieur de la partie de zone (8) une image de détail (9) est produite, qui comporte une profondeur et/ou un angle de vue et/ou un type de représentation et/ou une information de profondeur autre que la projection (6) et **en ce que** l'image de détail (9) est représentée sur l'écran à l'intérieur de la partie de zone (8).

2. Procédé selon la revendication 1,
**caractérisé en ce que**
l'image de détail est produite en retour direct ou indirect sur les données-images (1) à partir desquelles la projection est produite, sachant que ces données-images (1) sont réunies dans un premier jeu de données.

3. Procédé selon l'une quelconque des revendications précédentes,
**caractérisé en ce qu'**
une image de détail (9) est une sous-projection (10), qui se différencie des projections (6) par une profondeur de champ plus élevée.

4. Procédé selon la revendication 3,
**caractérisé en ce que**
le plan (4) des sous-projections (10) est parallèle au plan de la projection (6).

5. Procédé selon l'une quelconque des revendications précédentes,
**caractérisé en ce qu'**
une fenêtre est ouverte sur l'écran, dans laquelle différentes coupes sont représentées par l'objet (7) dans le cadre de la partie de zone (8) sélectionnée.

6. Procédé selon l'une quelconque des revendications précédentes,
**caractérisé en ce qu'**
une représentation de volume ou une représentation de surface a lieu dans la fenêtre séparée.

7. Procédé selon l'une quelconque des revendications précédentes,
**caractérisé en ce que**
lors de la production des sous-projections (10) avec une profondeur de champ plus élevée moins de plans d'images (4) sont superposés que lors de la production des projections (6).

8. Procédé selon l'une quelconque des revendications précédentes,
**caractérisé en ce qu'**
un plan d'image (4) représente exactement une sous-projection (10).

9. Procédé selon l'une quelconque des revendications précédentes,
**caractérisé en ce que**
l'utilisateur a accès interactif aux informations en images à l'intérieur de la partie de zone (8) dans laquelle il peut feuilleter parmi les différentes couches parallèlement aux plans de projection par déplacement d'un instrument de pointage.

10. Procédé selon l'une quelconque des revendications précédentes,
**caractérisé en ce que**
les données-images représentent une partie d'un corps humain ou animal et sont enregistrées avec un système de diagnostic.

11. Procédé selon la revendication 10,
**caractérisé en ce que**
les données-images sont enregistrées avec un tomodensitomètre assisté par ordinateur (TAO), un tomodensitomètre à résonance magnétique (IRM) ou par tomodensitométrie volumétrique numérique (TVN).

12. Procédé selon la revendication 10,
**caractérisé en ce que**
les données-images sont enregistrées avec un système à arceau en C qui a été tourné autour de l'objet.

13. Procédé selon l'une quelconque des revendications précédentes,
**caractérisé en ce que**
l'image de détail est produite en retour direct ou indirect aux données-images, qui sont réunies dans un deuxième jeu de données, sachant que ces données-images proviennent d'une autre prise de vue de l'objet.

14. Procédé selon la revendication 13,
**caractérisé en ce que**
les données-images du deuxième jeu de données ont été enregistrées avec un autre appareil, à un autre moment ou avec d'autres paramètres d'appareil.

15. Système de mise en œuvre du procédé selon l'une quelconque des revendications précédentes,
**caractérisé par**
un ordinateur avec accès aux données-images, sachant que sur l'ordinateur un programme est réalisé qui extrait des vues en coupe à partir des données-images et les superpose à la projection (6),
un écran sur lequel peut être représentée la projection (6),
des moyens, qui donnent à l'utilisateur la possibilité de définir à l'intérieur de la projection (6) une partie de zone (8), sachant que le programme comprend une fonction, qui produit une image de détail (9) en retour indirect ou direct aux données-images (1), sachant que l'image de détail (9) comporte une profondeur et/ou un angle de vue et/ou un type de représentation et/ou une information de profondeur, une teneur en informations autre que la projection (6) et est représentée sur l'écran à l'intérieur de la partie de zone (8).

16. Système selon la revendication 15,
**caractérisé par**
des moyens avec lesquels l'utilisateur sélectionne une image parmi différentes images de détail (9).

17. Utilisation du procédé selon l'une quelconque des revendications précédentes pour la présentation de données-images dans le domaine de l'odontologie.
